(19) Europäisches Patentamt
European Patent Office
Office européen des brevets

(11) Veröffentlichungsnummer: **0 279 436 B1**

# (12) EUROPÄISCHE PATENTSCHRIFT

(45) Veröffentlichungstag der Patentschrift: **02.01.91 Patentblatt 91/01**

(51) Int. Cl.$^5$: **C07C 43/196**

(21) Anmeldenummer: **88102330.3**

(22) Anmeldetag: **18.02.88**

## (54) 3-tert.-Butyl-4-methoxi-cyclohexylmethanol, dessen Herstellung und Verwendung als Riechstoff.

(30) Priorität: **19.02.87 DE 3705299**

(43) Veröffentlichungstag der Anmeldung: **24.08.88 Patentblatt 88/34**

(45) Bekanntmachung des Hinweises auf die Patenterteilung: **02.01.91 Patentblatt 91/01**

(84) Benannte Vertragsstaaten: **CH DE ES FR GB LI NL**

(56) Entgegenhaltungen:
**EP-A- 0 183 970**
**DE-A- 2 824 976**
**FR-A- 1 294 932**

(56) Entgegenhaltungen:
**THE JOURNAL OF ORGANIC CHEMISTRY, Band 46, 1981, Americal Chemical Society, Books and Journals Division; SEMIRAMIS AYRAL-KALOUSTIAN et al.: "Prerparation of 3-Hydroxy-cyclohexaneacetonitriles." Seiten 4880-4885**

(73) Patentinhaber: **BASF Aktiengesellschaft
Carl-Bosch-Strasse 38
D-6700 Ludwigshafen (DE)**

(72) Erfinder: **Degner, Dieter, Dr.
Kurpfalzstrasse 8
D-6701 Dannstadt-Schauernheim (DE)**
Erfinder: **Gramlich, Walter, Dr.
Auf der Hoehe 11
D-6803 Edingen-Neckarhausen (DE)**
Erfinder: **Schuster, Ludwig, Dr.
Weinheimer Strasse 44
D-6703 Limburgerhof (DE)**

**EP 0 279 436 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft 3-tert.-Butyl-4-methoxicyclohexylmethanol (I), enthaltende Duftstoffkompositonen sowie Herstellung

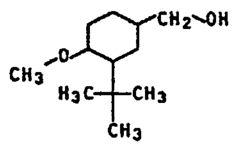

(I)

und Verwendung von I als Riechstoff.

Wegen der im allgemeinen unzureichenden Verfügbarkeit vieler natürlicher Riechstoffkomponenten, der notwendigen Anpassung an wechselnde modische Geschmacksrichtungen sowie dem ständig steigenden Bedarf an Geruchsverbesserern für Produkte des täglichen Bedarfs, wie Reinigungsmitteln, Kosmetika, leimen etc. hat die Riechstoffindustrie ständig Bedarf an neuen Riechstoffen, die allein oder in Form von Kompositionen wertvolle Parfüms bzw. Duftstoffe mit interessanten Duftnoten darstellen. Da aufgrund wenig bekannter Zusammenhänge zwischen Struktur und Riechstoffeigenschaften eine gezielte Synthese von Riechstoffen mit gewünschten olfaktorischen Eigenschaften nicht möglich ist, besteht die Aufgabe, solche Verbindungen aufzufinden, die wertvolle Riechstoffqualitäten besitzen.

Es war daher die Aufgabe der Erfindung, neue interessante Riechstoffe zu entwickeln, die auf möglichst einfache Weise aus gut zugänglichen und daher billigen Ausgangsstoffen hergestellt werden können.

Mit der erfindungsgemäßen Verbindung I wurde nun eine neue Verbindung gefunden, die ganz überraschend eine extrem warme und langhaftende Moschus-Note aufweist.

Verbindungen mit einer Moschus-Note gehören in der Parfümerie seit frühester Zeit zu den wertvollsten Gerüchsklassen.

Während in früheren Jahrhunderten der Moschus-Duft fast ausschließlich aus der Drüse des Moschus-Tieres (Moschus moschiferus Linnaeus) gewonnen wurde, ist diese Tinktur heute praktisch unbezahlbar und wurde ab Anfang des 20. Jahrhunderts (1888 stellte A. Baur die erste synthetische Moschus-Verbindung her) fortlaufend durch synthetische Produkte ersetzt.

Die Bedeutung des Moschus-Duftes geht allein daraus hervor, daß heutzutage praktisch kein erfolgreiches Parfüm komponiert wird, ohne die Verwendung von Moschus-Verbindungen. Das Buch : 'Fragrance Chemistry', The Science of the Scence of Smell, E. T. Theimer, Academic Press 1982, gibt auf 100 Seiten (S. 433 - 534) einen ausgezeichneten Überblick über die derzeit bekannten, unterschiedlichen Substanzklassen mit Moschus-Duft.

Man kann allgemein Moschus-Verbindungen in drei große Substanz-Klassen unterteilen :

1. Makrocyclische Verbindungen, zumeist Ketone, Lactone oder Pyridine wie z.B. Exalton (2), Muscon (3), Ambrettolid (4) oder der Pyridin-Moschus (5).

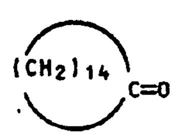

2

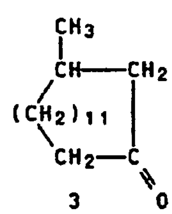

3

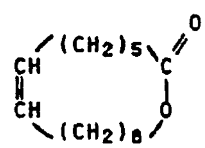

4

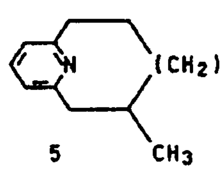

5

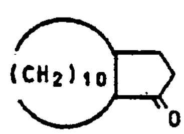

6

Ebenso können diese Makrocyclen als Bicyclen wie z.B. (6) vorkommen.

2. Aromatische mono- und bicyclische Nitro-Verbindungen. Kommerziell bedeutende Verbindungen

sind

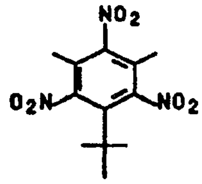 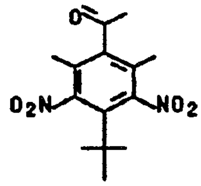 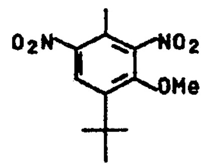 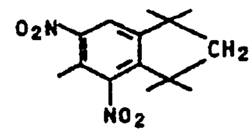

7 8 9 10

Musk Xylol (7), Musk Keton (8), Musk Ambrette (9) sowie Mosken (10).

3. Bi- und tricyclische benzoide Verbindungen. Kommerziell bedeutende bicyclische Verbindungen sind

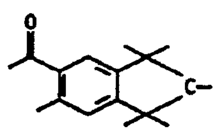 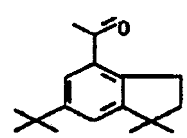 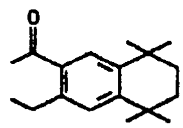 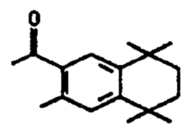

11 12 13 14

Phantolid® (Polaks's Frutal Works ; 11), Celestolide® (JFF ; 12),Versalide® (Givaudan ; 13) sowie Tonalid (PFW ; 14), die bedeutendsten tricyclischen Verbindungen sind

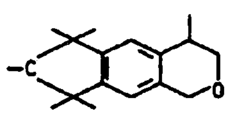 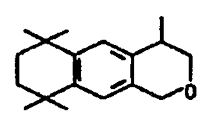

15 16

Galaxalid (15) und Musk 89 (16) der Firma JFF.

Die bekannten synthetischen Moschusriechstoffe sind allgemein nur durch aufwendige Verfahren herstellbar.

Es war daher völlig überraschend, daß die neue Verbindung I, also eine Verbindung mit einer völlig anderen Struktur, die zudem auf einfache Weise aus gut zugänglichen und daher billigen Ausgangsstoffen hergestellt werden kann, einen warmen und langhaftenden Moschusduft aufweist.

Strukturell verwandte Cyclohexylmethanole haben als Riechstoffe wirtschaftlich nur geringe Bedeutung, wobei die einzige Ausnahme 4-Isopropyl-cyclohexylmethanol (17) darstellt,

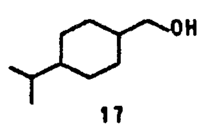

17

die unter dem Markennamen Mayol® (Firmenich) vertrieben wird ; Mayol® weist einen Maiglöckchen-Duft auf, d.h. eine von der erfindungsgemäßen Verbindung völlig unterschiedliche Duftrichtung.

Die Herstellung der erfindungsgemäßen Verbindung erfolgt auf an sich bekannte Weise durch katalytische Hydrierung von 3-tert.-Butyl-4-methoxi-benzaldehyd (18), der sich leicht aus

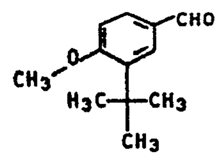

18

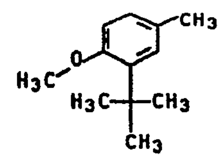

19

4-Methyl-2-tert.-butyl-anisol (19) durch Oxidation mit Mangan (IV) oxid nach bekannten Verfahren (US-PS 24 50 877 bzw. US-PS 24 50 878) erhalten läßt.

Für die Kernhydrierung sowie die gleichzeitige Reduktion der Aldehyd-Gruppe können die bekannten Nickel-, Palladium-, Rhodium- oder Ruthenium-Katalysatoren, vorzugsweise Rutheniumkatalysatoren, eingesetzt werden.

Man arbeitet im allgemeinen bei Temperaturen von 50 bis 250, vorzugsweise 100 bis 200°C und Wasserstoffdrücken von 30 bis 500, vorzugsweise 100 bis 300 bar.

Als Lösungsmittel für die Hydrierung können beispielsweise Alkanole, wie Methanol oder Ethanol ; Ether, wie Tetrahydrofuran ; Kohlenwasserstoffe, wie Pentan ; und Säuren wie Essigsäure eingesetzt werden.

Das in Gegenwart von Rutheniumkatalysatoren erhaltene Produkt stellt nach $^{1}H$- und $^{13}C$-NMR-spektroskopischen Untersuchungen ein Diastereomerengemisch dar, in dem das Isomere, in dem alle drei Substituenten in cis-Stellung stehen, überwiegt, d.h. einen Anteil von über 90 % ausmacht (siehe Beispiel).

Die nach destillativer Reinigung erhaltene Verbindung I weist einen warmenMoschus-Duft mit großer Haftdauer auf. Sie stellt ein farbloses Öl dar, das in Wasser praktisch unlöslich, aber in den bekannten organischen Lösungsmitteln gut löslich ist.

Aufgrund der beschriebenen Geruchseigenschaften kann I vorteilhaft als Riechstoff oder Bestandteil von Riechstoffkompositionen und Parfümölen für kosmetische und technische Anwendungen wie Reinigungs- und Pflegemittel für den Haushalt, d.h. also zur Verleihung, Verbesserung oder Modifizierung der Dufteigenschaften von Parfüms oder parfümierten Produkten eingesetzt werden. Die neue Verbindung läßt sich ausgezeichnet mit anderenRiechstoffen kombinieren und verleiht ihnen eine größere Haftfestigkeit ; darüber hinaus ist sie als gesättigter Alkohol in einem weiten pH-Bereich stabil, so daß sie breit angewendet werden kann. Als weiterer Vorteil ist zu nennen, daß die neue Verbindung I - im Gegensatz zu den meisten marktgängigen Moschus-Riechstoffen, die Feststoffe sind - flüssig ist, was eine bessere Verarbeitung gewährleistet.

Beispiel

Herstellung von 3-tert.-Butyl-4-methoxi-cyclohexylmethanol

In einem Autoklaven wurde ein Gemisch aus 300 g (1,56 mol) 3-tert.-Butyl-4-methoxi-benzaldehyde, 300 ml Tetrahydrofuran und 3 g Rutheniumhydroxid $[RuO(OH)_x]$ bei 150°C und 300 bar Wasserstoffdruck hydriert. Nach zwei Stunden war die Hydrierung beendet und man ließ abkühlen.

Nach Abtrennen des Katalysators und Entfernen des Lösungsmittels wurde der Rückstand unter vermindertem Druck fraktioniert. Nach einer kleinen Vorlauf-Fraktion erhielt man 274 g (entsprechend 88 % Ausbeute) eines Diastereomerengemisches an 3-tert.-Butyl-4-methoxi-cyclohexylmethanol, in dem das Isomere bei dem alle drei Reste cis-Stellung aufweisen, mit über 90 % dominiert, in Form eines farblosen Öls mit den nachfolgend aufgeführen physikalischen Eigenschaften.

Kp. 79°C/0,2 mbar

$n^{2}β$ 1,4732

IR (Film) : 3336, 2950, 2931, 2867, 2820, 1479, 1361, 1090, 1056, 1032 $cm^{-1}$.

$^{1}H$-NMR ($CDCl_3$) : $\delta$ = 0,93(s,9H), 1-1,3(m,4H), 1,42-1,65(m,3H), 2,1(m,1H), 2,4(br.s,1H,-OH), 3,28(s,3H), 3,47(d,2H), 3,62(s,1H)ppm.

$^{13}C$-NMR ($CDCl_3$) (Bezeichnung siehe Formel) :

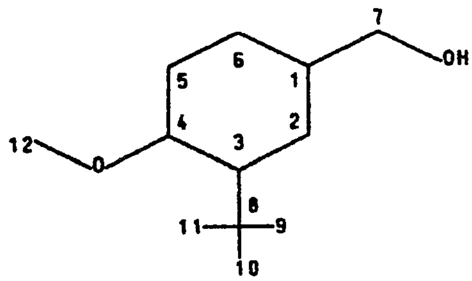

δ = 76,84($C_4$,d), 68,47($C_7$,t), 55,34($C_{12}$,q), 50,62($C_3$,d), 40,91($C_1$,d), 32,67($C_8$,s), 28,72($C_9$,$C_{10}$,$C_{11}$ ;q), 27,87($C_5$,t),25,20($C_6$,t), 23,20($C_2$,t) ppm.

MS (m/l) : 200 ($M^+$, 20 %), 182 (5), 168 (27), 153 (7), 135 (20), 124 (48), 109 (50), 94 (88), 79 (77), 71 (100), 67 (45), 57 (90), 41 (89).

Anwendungsbeispiel

Durch Hinzufügen von 10 Teilen 3-tert.Butyl-4-methoxi-cyclohexylamethanol zu der folgenden Blumen-Base (vgl. Perfumery Technology, F.W. Wells und M. Billot, John Wiley & Sons 1981, S. 276) :

| | |
|---|---|
| Anisaldehyd | 40 Gew.-Teile |
| Anisalkohol | 5 Gew.-Teile |
| Acetophenon | 1 Gew.-Teil |
| Methylacetophenon | 3 Gew.-Teile |
| Hydroxicitronellal | 8 Gew.-Teile |
| Zimtalkohol | 9 Gew.-Teile |
| Citronellol | 8 Gew.-Teile |
| Heliotropin | 2 Gew.-Teile |
| Cumarin | 5 Gew.-Teile |
| Linalool | 3 Gew.-Teile |
| 2-Phenylethanol | 5 Gew.-Teile |
| Geraniol | 6 Gew.-Teile |
| Terpineol | 5 Gew.-Teile |
| | 100 Gew.-Teile |

wurde eine interessante Abrundung des Dufts mit einer warmen Moschus-Note und größerer Haftdauer erzielt.

**Ansprüche**

1. 3-tert.-Butyl-4-methoxi-cyclohexylmethanol.
2. Duftstoffkompositionen, enthaltend 3-tert.-Butyl-4-methoxicyclohexylmethanol.
3. Verwendung von 3-tert.-Butyl-4-methoxicyclohexylmethanol zur Verleihung, Verbesserung oder Modifizierung der Dufteigenschaften von Parfüms oder parfümierten Produkten.
4. Verfahren zur Herstellung von 3-tert.-Butyl-4-methoxicyclohexylmethanol, dadurch gekennzeichnet, daß man 3-tert.-Butyl-4-methoxi-benzaldehyd hydriert.

## Claims

1. 3-tert.-butyl-4-methoxycyclohexylmethanol.

2. A fragrance composition containing 3-tert.-butyl4-methoxycyclohexylmethanol.

3. Use of 3-tert.-butyl-4-methoxycyclohexylmethanol for imparting fragrance properties to perfumes or perfume products or for improving or modifying said properties of perfumes or perfume products.

4. A process for the preparation of 3-tert.-butyl4-methoxycyclohexylmethanol, wherein 3-tert.-butyl-4-methoxybenzaldehyde is hydrogenated.

## Revendications

1. 3-tert.-Butyl-4-méthoxy-cyclohexylméthanol.

2. Compositions odorantes contenant du 3-tert.butyl-4-méthoxy-cyclohexylméthanol.

3. Utilisation du 3-tert.-butyl-4-méthoxy-cyclohexyl-méthanol pour améliorer, perfectionner ou modifier les propriétés aromatiques de parfums ou de produits parfumés.

4. Procédé de préparation de 3-tert.-butyl-4-méthoxycyclohexylméthanol, caractérisé en ce qu'on hydrogène du 3-tert.-butyl-4-méthoxybenzaldéhyde.